# EUROPEAN PATENT SPECIFICATION

(11) **EP 0 864 649 B1**
(45) Date of publication and mention of the grant of the patent: **26.04.2006**
(21) Application number: 98202021.6
(22) Date of filing: 07.10.1991
(51) Int. Cl.: C12N 15/51, C07K 14/02, A61K 39/29, C12N 1/19, C12N 5/12, C07K 16/08

(54) **Method for obtaining recombinant surface antigen of hepatitis B virus (HEP B) of higher immunogenic capacity and use thereof in a vaccine preparation**
Verfahren zur Herstellung von recombinantem Hepatitis B Oberflächenantigen mit erhöhter Immunogen-Kapazität und Verwendung in einer Impfstoffzubereitung
Méthode de préparation de l'antigène de surface de l'hepatite B avec une capacité immunogénique supérieure et utalisation d'une préparation de vaccin

(30) Priority: 08.10.1990 CU 15590
(43) Date of publication of application: 16.09.1998
(62) Divisional of application: 91202615.0
(73) Proprietor: CENTRO DE INGENIERIA GENETICA Y BIOTECNOLOGIA, Havana (CU)
(72) Inventor: Muzio, González, Verena L., Plaza de la Revolucion,Ciudad d.l.Habana (CU); Penton Arias, Eduardo, Ciudad de la Habana (CU); Fontirrochi Escobar, Giuvel, Edén, Camagüey (CU); Nazábal Gálvez, Marcelo, Puentes Grandes, Playa. C. de la Habana (CU); González Griego, Marta de Jesus, Lawton, 10 de Octubre, C. de la Habana (CU); Beldarrain Iznaga, Alejandro, Marianao, Ciudad de la Habana (CU); Padron Gonzalez, Guillermo Julio, Cubanacan Playa, Ciudad de la Habana (CU); Ramirez Albajes, Victoria, Playa Ciudad de la Habana (CU); Garcia Pena, Arnaldo, Ciudad de la Habana (CU); Ruiz Cruz, Carlos Enrique, Ciudad de la Habana (CU); Izquierdo Lopez, Gladys Mabel, Vibora, 10 de Oct., Ciudad de la Habana (CU); Herrera Martinez, Luis Saturnino, Ciudad de la Habana (CU); Duarte Cano, Carlos Antonio, Center Habana, Ciudad de la Habana (CU); Pérez Suarez, Lilia Luisa, Playa, Ciudad de la Habana (CU); Garcia Suárez, José, Plaza C.de la Habana (CU); de la Riva de la Riva, Gustavo Alberto, Vibora, Ciudad de la Habana (CU); Santiago Avila, Ramon Alexis, Lisa, Ciudad de la Habana (CU); Ayon Zorrilla, Filiberto Rosendo, Playa, Ciudad de la Habana (CU); Paez Meireles, Rolando, La Habana (CU); Agraz Fierro, Alberto, Playa, Ciudad de la Habana (CU); Diaz Betancourt, Raul Enrique, Playa, Ciudad de la Habana (CU); Quinones Maya, Yair, Playa, Ciudad de la Habana (CU)
(74) Representative: Smulders, Theodorus A.H.J.

(56) References cited:
- EP-A- 0 337 492

## Description

The present invention is in the field of genetic engineering and biotechnology and in particular that of the production of vaccines against hepatitis B virus.

Infection with the hepatitis B virus is an important health problem on a worldwide scale, since not only does it cause acute illness, but also it can evolve into chronic forms, liver cirrhosis and insufficiency, and hepatocellular carcinoma. It is calculated that in the world there are about 200 million chronic carriers of the virus, who constitute the principal reservoir of this infectious agent.

At present there is no effective treatment against the virus, so that prevention is the only way to reduce its spread and lower the incidence of infection.

There are two main strategies for the production of vaccines against hepatitis B virus: those derived from plasma and those obtained by genetic engineering techniques.

The vaccines derived from plasma are based on purification of HBsAg from the plasma of chronic carriers of the virus by various physical methods and inactivation thereof (A.J. Zuckerman et al., British Medical Journal, 1985, 290 : 492). These vaccines have demonstrated their efficacy and safety for years (A.M. Prince et al., Annual Clinical Research, 1982, 14: 225) and have not been associated with the risk of transmission of human immunodeficiency virus or other infectious agents (F. Deinhardt et al., Journal of Medical Virology, 1985, 17: 209).

However, extensive use of this vaccine is affected by the limited availability of sera of carriers of the virus for obtaining HBsAg used for production of the vaccine, the need for strict procedures for the purification and inactivation of hepatitis B virus and removal of other infectious agents which may be present in the plasma, as well as the prolonged safety tests necessary for clearance of the batches of vaccines. Moreover there is the particular fear that infectious agents which cause blood-transmissible diseases may escape the process of inactivation during production of the vaccine, on account of which there is a general tendency to replace blood-derived products with those obtained by genetic engineering techniques.

The hepatitis B virus surface antigen gene has been cloned and expressed in prokaryotic and eukaryotic cells.

In bacteria, the levels of expression obtained have been low, and efficient incorporation of the antigen in immunogenic particles of 22 nm does not take place, which is why this system has not been used as an antigen source for the production of vaccines (P. Valenzuela et al., Nature, 1980, 280: 815; C.J. Burrell et al., Nature, 1979, 279: 43).

Eukaryotic cells have also been used in the production of HBsAg (M.L. Michel et al., Biotechnology, 1985, 3 : 561; G.M. MacNab et al., British Journal of Cancer, 1976, 36: 509). However, the production of vaccines using this method requires complex and costly equipment, methodology and culture media, and poses problems in the scaling up of production. There are also fears relating to the safety of vaccines derived from mammalian cell lines which are tumorigenic due to the risk of the presence of retrovirus, and so although the antigen obtained has characteristics similar to the natural antigen, this method is not used either for mass production of this vaccine.

The genetically manipulated vaccinia virus makes it possible to obtain recombinant viruses which express HBsAg alone or in combination with other antigens for the production of live vaccines against infectious agents (C. Cheng et al., Journal of Virology, 1986, 60: 337; E. Paoletti et al., Proceedings of the National Academy of Sciences USA, 1984, 81: 193). The vaccines obtained in this way have not been approved for large-scale use in human beings because of numerous technical and ethical considerations.

The recombinant vaccines against hepatitis B which are available commercially are fundamentally based on the production of HBsAg by genetically manipulated yeasts.

The yeast Saccharomyces cerevisiae has been widely used for the production of biologically active heterologous proteins for wide use (S.M. Kingsman et al, Tictech, 1987, 5 : 53) and for obtaining HBsAg in large quantities (W.J. McAleer et al., Nature, 1984, 307: 178; N. Harford et al., Postgraduate Medical Journal, 1987, 63 suppl. 2: 65; G.A. Bitter et al., Journal of Medical Virology, 1988, 25: 123).

The antigen obtained is produced intracellularly and is extracted by different methods of cellular disruption and purified by various physicochemical methods which have made it possible to achieve levels of purity greater than 97% and with a product which has an antigenic behaviour similar to the plasma antigen as demonstrated in animals and human beings (P. Hauser et al., Postgraduate Medical Journal, 1987, 63 suppl. 2: 83).

Recently an efficient system of expression has been reported which uses as the host the methylotrophic yeast Pichia pastoris and which is based on use of the promotor of the gene for the enzyme Alcohol Oxidase I, which is the first enzyme to be involved in the pathway of utilization of methanol by this yeast; said promotor is strictly regulated and makes it possible to express high levels of the enzyme (up to 30%) when the cells grow in the presence of methanol and not when they grow in the presence of glucose (S.B. Ellis et al., Molecular and Cellular Biology, 1985, 5: 1111; R. Couderc et al., Agric. Biol. Chem., 1980, 44: 2259).

The yeast P. pastoris has already been used for the expression of various heterologous proteins, including HBsAg (J.M. Cregg et al., Biotechnology, 1987, 5: 479), in a genetic construct in which the gene which codes for HBsAg is cloned under the regulation signals of the promotor of the enzyme Alcohol Oxidase I, in an expression cassette which is integrated in the chromosome of a mutant strain of this yeast, which makes it possible, on activation of the system in the presence of methanol, for 2-3% of the soluble protein produced to be HBsAg. A purification of the antigen is not disclosed, however.

An important advantage which the Pichia pastoris system offers with respect to that of S. cerevisiae is the very efficient incorporation of the antigen into particles of 22 nm, since virtually all the antigen produced is in particulated form, unlike what happens in S. cerevisiae in which only a small proportion of the monomer of 24 kDa is incorporated into the antigen particles (P. Valenzuela et al., Nature, 1982, 298: 347; R.A. Hitzeman et al., Nucleic Acids Research, 1983, 11: 2745; A. Miyanohara et al., Proceedings of the National Academy of Sciences USA, 1983, 80: 1). Nevertheless, the vaccine preparations based on recombinant HBsAg currently available on the market continue to use the yeast S. cerevisiae as a source of it.

On the other hand, the most commonly used purification methods of Hepatitis B surface antigen from yeast involved the use of methods of density gradient centrifugation (Smith Kline's European patent application No. 278 940 A3) which are expensive and complex to carry out on an industrial scale.

In the Phillips Petroleum's European patent application No. 337 492 is described a process for recovering Hepatitis B surface antigen from a recombinant strain of Pichia pastoris. Although the purpose is to recover the hepatitis B surface antigen in a state of purity sufficient to be incorporated directly into a vaccine, neither the characteristics of the antigen in relation to its particulation after the purification, nor its immunogenic capacity when it is included in a vaccine preparation are described.

The Phillips Petroleum process comprises lysing the yeast cells in the presence of a chaotropic salt and separating the supernatant which contains the antigen from the lysed cell pellet; precipitating lipids and contaminating proteins from the supernatant at a pH of 4.5-5.5 and removing the precipitated residue; subjecting the supernatant to concentration and diafiltration treatments; contacting the retentate which contains the antigen with silica, washing contaminant proteins from the silica and eluting the hepatitis B surface antigen with a buffer having a pH of 9.5-11.0 which contains urea; subjecting the appropriate fraction to gel filtration with a material having a molecular weight exclusion limit suitable to separate the hepatitis B surface antigen particle from contaminants; contacting the appropriate fraction with an anion exchange resin and eluting the hepatitis B surface antigen particle from the resin with a buffer having a pH of 6-9.

In this process a chaotropic buffer is used for the lysis/extraction step. This buffer contains potassium thiocyanate as the chaotropic agent and phenyl methyl sulphonyl fluoride as protease inhibitor. This last compound is highly poisonous, so it is necessary to eliminate all traces of it during the purification process. Furthermore, even though this substance is effective in the protease inhibition, its spectrum of inhibition is limited.

Afterwards, the supernatant containing the HBsAg is diafiltrated and this fraction is contacted with silica. In order to carry out the separation of hepatitis B surface antigen from the silica, an urea-containing buffer with a pH in the range of 9.5 to 11 is employed. This compound is highly denaturalising and its use can lead to a loss of immunogenicity of the obtained product.

The present invention provides a new recombinant hepatitis B surface antigen, a new vaccine composition for immunizing againt Hepatitis B virus, and a new use of a particular Pichia pastoris strain and anti-HBsAg monoclonal antibody, all as defined in the claims. The description discloses a process of purification guaranteeing to obtain a recombinant antigen from Pichia pastoris which is characterised by a high degree of particulation and homogeneity, which confers superior immunogenic characteristics upon it.

The said process for recovering hepatitis B surface antigen from Pichia pastoris cells containing a gene encoding said, hepatitis B surface antigen and having expressed it, comprises growing cells of Pichia pastoris strain C226 [pTAO906], CBS450.90, containing a gene encoding said hepatitis B surface antigen under conditions resulting in its expression; lysing the cells in the presence of a buffer comprising a chaotropic agent, sucrose, and ethylene diamino tetra acetic acid (EDTA) ;
precipitating contaminants at an acid pH;
subjecting the antigen preparation to an acid adsorption and alkaline desorption treatment on a matrix of diatomaceous earth;
subjecting the antigen preparation to immunoaffinity chromatography using the hepatitis B surface antigen-specific monoclonal antibody CB HEP 1 produced by hybridoma cell line ECACC 90 112 606;
subjecting the eluted antigen to a heat treatment at a temperature of 30-40°C;
washing the antigen in an anion exchange column with detergent; and subjecting the eluted antigen to HPLC in the presence of a detergent.

It is preferred that the lysis of the cells is carried out in the presence of a buffer comprising potassium thiocyanate, sucrose, EDTA, Tris and NaCl. Most preferably, the lysis of the cells is carried out in the presence of a buffer comprising 1-4 M potassium thiocyanate, 1-15% (w/v) sucrose, 2.5-3.5 mM EDTA, 10-30 mM Tris and 0.1-1 M NaCl, preferably essentially consisting of 291 g/l potassium thiocyanate, 100 g/l sucrose, 1.86 g/l EDTA, 12 g/l Tris and 17.5 g/l NaCl.

It is also preferred that the acid precipitation of the contaminants is carried out at a pH of 3-4.

If desired, the antigen preparation obtained after removal of the precipitate formed in the acid precipitation step may be stored at a pH of 7-8.

Preferably, the antigen preparation obtained after removal of the precipitate formed in the acid precipitation step is contacted with a matrix of diatomaceous earth at a pH of 3-5 to adsorb the antigen and contaminants are eluted with an elution buffer having a pH of 3-5, followed by desorption of the antigen at a pH of 7.5-9.0.

If desired, the antigen preparation desorbed from the diatomaceous earth may be subjected to conventional concentration and desalting treatments, e.g. concentrated by ultrafiltration and desalted by diafiltration.

The monoclonal antibody used in the immunoaffinity chromatography treatment of the antigen preparation is a monoclonal antibody selected for a high affinity to antigenic hepatitis B surface antigen particles. The monoclonal antibody used in the immunoaffinity chromatography treatment of the antigen preparation is anti HBsAg CB-HEP1 produced by hybridoma cell line ECACC 90 112 606.

Preferably, the antigen is eluted from the immunoaffinity chromatography column with a buffer containing a chaotropic agent.

It is preferred that the heat treatment of the eluted antigen at 30-40°C is carried out for 1-6 hours.

After the heat treatment, the antigen is washed preferably in a DEAE-cellulose column with a detergent such as sodium deoxicolate and Triton X-100. The detergent is used in a concentration of 0.01-0.5% by weight, preferably 0.05-0.1% by weight. Then the antigen is eluted.

The eluted antigen is then preferably subjected to HPLC with cut-off between 20,000 and 10,000,000 in the presence of a detergent such as sodium deoxicolate. The detergent is used in a concentration of 0,01-0.5% by weight, preferably about 0.05% by weight.

The hepatitis B surface antigen is recovered from the recombinant Pichia pastoris strain C226 [pTAO906], CBS450.90.

The invention will now be explained in more detail.

The first step of the process is to lyse the cells with a buffer containing EDTA, Tris, NaCl, sucrose and potassium thiocyanate. In this step is important the presence of EDTA which is a protease inhibitor with a wide spectrum of inhibition and also it is totally biocompatible. The sucrose maintains the conformational state of the molecule as well as its half life. Potassium thiocyanate is a conventional chaotropic agent; others may be used instead.

After the rupture of the cells, the separation of the debris is carried out simultaneously with an acid precipitation of contaminant proteins, using a pH range of 3 to 4. This operation allows to reduce the number of steps during the purification process and at the same time to start the process with a material of high purity (more than 10% of antigen) and with good recovery. Furthermore, this step assures the elimination of contaminant lipids, carbohydrates and DNA. After centrifugation, the supernatant containing the antigen may be stored at a pH between 7 and 8. In order to carry out the subsequent purification, the pH of the preparation is lowered again to a pH between 3 and 5 and then the preparation is allowed to contact a matrix of Celite where the antigen is adsorbed. The matrix is washed with a buffered solution having a pH between 3 and 5 (Tris-HCl, potassium thiocyanate, sucrose and EDTA) to remove contaminants such as carbohydrates, lipids and DNA. The antigen is recovered from the matrix by a change of pH, using a buffer with pH between 7.5 and 9.0. This step assures that the antigen is recovered with a purity between 40 and 50%, without risks of denaturalisation or loss of particularity of the antigen because the gentle conditions employed during its elution.

The eluant containing the antigen desorbed from the Celite is concentrated between 30 to 40 times by ultrafiltration using a membrane of 0,1 µm and later the preparation is diafiltrated with 3 volumens of an adequate buffer (Tris.HCl, EDTA, pH between 7 and 8) and then subjected to a further purification step by immunoaffinity chromatography, using monoclonal antibodies specially screened for the selection of antigenic particles. These monoclonal antibodies are used to avoid stressing treatments and possibly recognize specific epitopes having particular immunogenic properties as has been demonstrated in chimpanzees (Schellekens, H; De Reus, A; Peetermans, J.H. and Van Eerd, P.A.C.M. Postgraduated Medical Journal, 1987, 63, Supplement (2), p. 93-96). The fraction containing the HBsAg obtained from the previous step is applied in the affinity column equilibrated in an adequate buffer such as Tris-HCl, potassium or sodium phosphate with pH between 6 and 7 using a flow between 25 and 50 cm/h. The non adsorbed fractions in the column (contaminant substances) are washed using the equilibration buffer with an addition of 1 M of NaCl. The antigen is recovered from the column using a buffer such as Tris-HCl or phosphate containing potassium thiocyanate in a range between 1 and 3 M. The material obtained from this step has a purity of more than 85%.

Another novel aspect of this method is that the eluant containing the antigen obtained in the previous step is then subjected to a heat treatment at 30 to 40°C, preferably 37°C, during a time period of 1 to 6 hours. This step allows a high recovery of homogeneous particles of 22 nm, which display a high immunogenicity. Specifically, this fact is shown in the next step consisting of ion exchange in DEAE cellulose, in which step these particles elute in a strong peak with 175 mM NaCl, which fraction is different from the remainder fractions which contain particulated forms of the antigen with lower antigenic activity.

The eluted fraction containing the antigen is then desalted by gel filtration chromatography with an adequate buffer (Tris-HCl or phosphate buffer) in a pH range of 7 to 7.5. Subsequently, this material is applied onto an ion exchange column like DEAE cellulose and the resin is washed with different concentrations of NaCl in a range between 10 and 50 mM and detergents such as sodium deoxicolate and Triton X-100 to concentrations in a range between 0.05 and 0.1%. These washes assure the elimination of contaminant DNA and endotoxins. The antigen is recovered from the column using a solution of NaCl between 150 and 200 mM and potassium thiocyanate. This preparation is completely free of DNA and it has a purity level above 95%.

The eluted antigen is concentrated using an ultrafiltration system with membranes of cut-off of 100,000 daltons until a protein concentration in a range between 1 to 3 mg/ml. A detergent such as sodium deoxicolate is added in a concentration of 0.05% to the preparation and then the concentrate was subjected to high resolution gel filtration chromatography with a work range between 10,000,000 and 20,000 daltons (PW 5000 or PW 6000). The column was equilibrated with a buffer containing 0.05% of sodium deoxicolate. The presence of detergent during the operation decreases the endotoxin level of the antigenic preparation to less than 0.4 ng/µg of antigen and allows the separation of non properly particulated fractions.

The antigen obtained is desalted again in a gel filtration column in an adequate buffer in order to be adjuvated in aluminum hydroxide gel in order to be used in a vaccine preparation.

Verification of the superior immunogenicity characteristics of the vaccine preparation obtained by use of the methods indicated, is based on the results of controlled preclinical tests carried out in accordance with a strict double-blind protocol.

By the method described herein it is possible to obtain a product of superior quality as an immunogen to others of the same kind available commercially for use in human beings.

### PRACTICAL EXAMPLES:

### EXAMPLE 1:

To obtain the hepatitis B virus surface antigen gene, the viral DNA was cloned in the EcoRI site of pBR322. The viral genome was obtained from Dane particles isolated and purified from the serum of an asymptomatic carrier of the virus by a method similar to the one reported (P. Valenzuela et al., Nature, 1979, 280: 815). The resulting plasmid, called pHBS1, was digested with the enzymes EcoRI and HpaI to obtain a fragment which in turn was digested with the enzyme TaqI, and in this site an EcoRI linker (5' GGAATTCC 3') was ligated and subcloned in the EcoRI site of pBR322. The resulting plasmid was called pHBS2, from which was obtained the fragment which contains the HBsAg gene by digestion with EcoRI and treatment with S₁ nuclease.

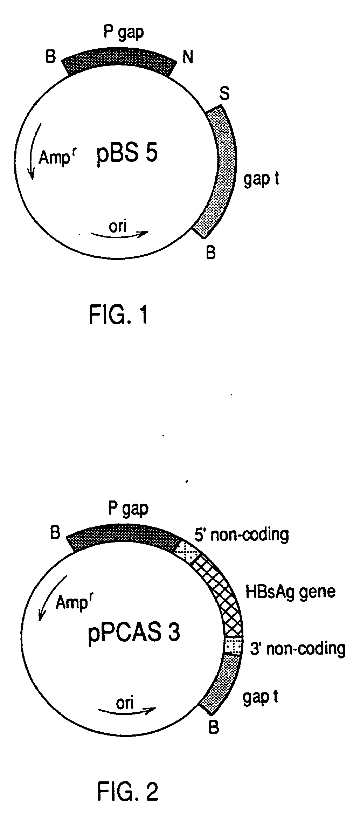

This fragment was subcloned in the vector pBS5 (Fig. 1) previously linearised with NcoI and treated with S₁ nuclease and alkaline phosphatase. The resulting plasmid was called pPCAS3 (Fig. 2), in which the non-coding viral regions at the 5' (25 bp) and 3' (125 bp) ends which flanked said gene were eliminated by the polymerase chain reaction (PCR) according to standard reaction conditions (R.K. Saiki et al., Science, 1985, 230 : 1350).

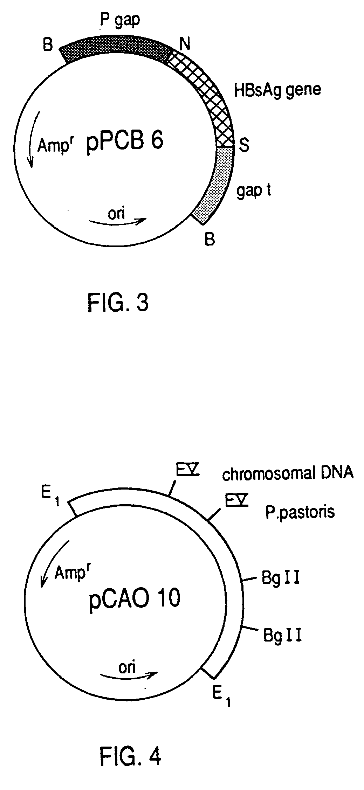

By using two synthetic oligonucleotides, the sequences of which are: 5' region: 5' GCCATGGAGAACATCACAT 3' (SEQ ID NO:1) 3' region: 3' GACCCATATGTAAATTTGCAGCTGG 5' (SEQ ID NO:2), NcoI and SalI restrictions sites were created at the 5' and 3' ends of the gene respectively, which allowed easy subsequent manipulation of it and elimination of the non-coding regions on digestion of the amplified fragment with the above-mentioned enzymes. The fragment of 678 bp obtained, which contains the exact HBsAg gene, was subcloned in the plasmid pBS5. The resulting plasmid, called pPCB6 (Fig. 3), contains said gene under the transcription regulation signals of the gene for the enzyme glyceraldehyde 3-phosphate dehydrogenase (GAP) of S. cerevisiae.

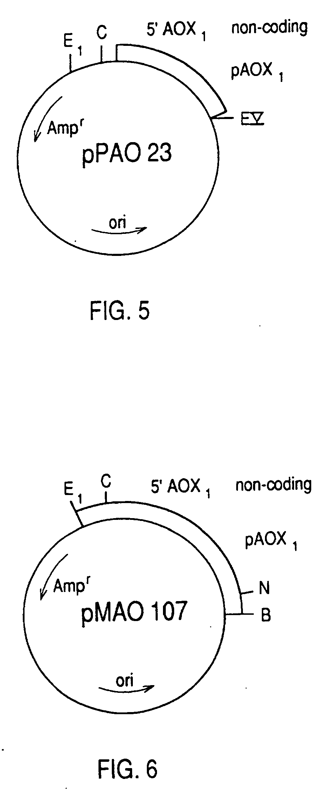

From the plasmid pCAO10, obtained from a DNA library of Pichia pastoris (Fig. 4), was extracted the 1.1 kb fragment which contains the promotor of the gene for the enzyme AOX1, as well as a non-coding 5' region, which was subcloned in plasmid PBR322, the resulting plasmid being pPAO23 (Fig. 5) on which was carried out the polymerase chain reaction technique for the purpose of eliminating 18 bp from the structural gene for the enzyme AOX1, which correspond to the codons which code for the first 6 amino acids of this protein which were present in this construct.

The oligonucleotides used were:

5' region: 5' GTATCACGAGGCCCT 3' (SEQ ID NO:3)

3' region: 3' TTGATTAATAAGCTTTGGTACCGC 5'    (SEQ ID NO:4).

The oligonucleotide of the 3' region allows the creation of an NcoI site at the 3' end of the promotor of the gene for the enzyme AOX1 in order to eliminate the sequence of 18 bp of the structural gene for this enzyme and achieve exact binding to the HBsAg gene. The oligonucleotide of the 5' region allows retention of the EcoRI site of PBR322.

The amplified fragment of 1115 bp was subcloned in the plasmid pUC19, resulting in the plasmid called pMAO107 (Fig. 6) which contains the perfect promotor of the gene for the enzyme AOX1 of P. pastoris.

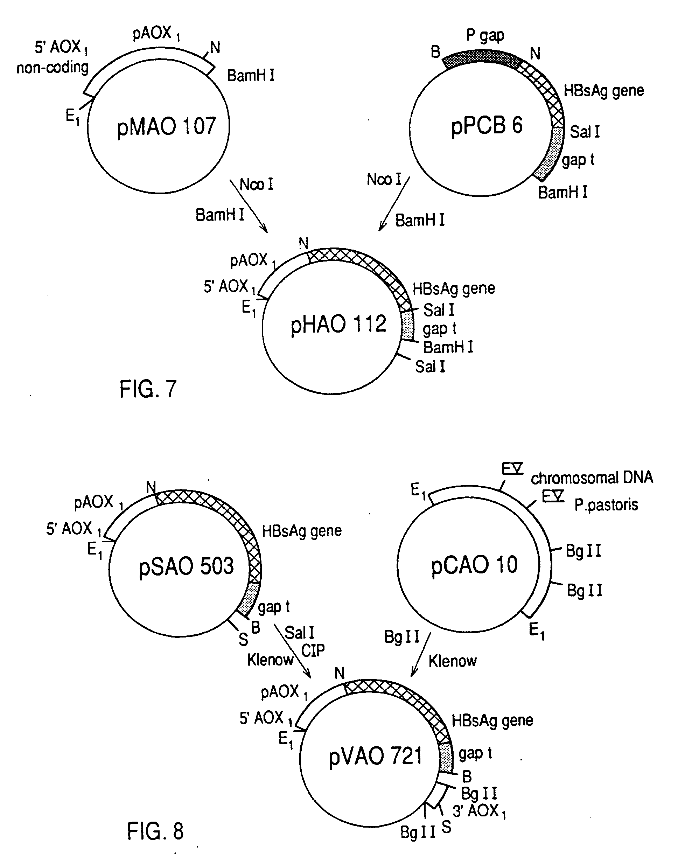

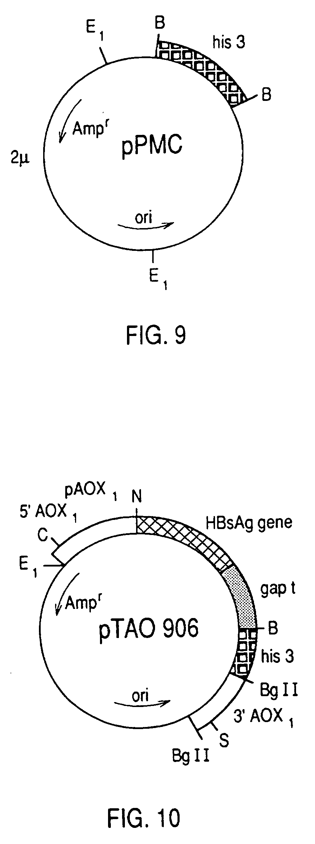

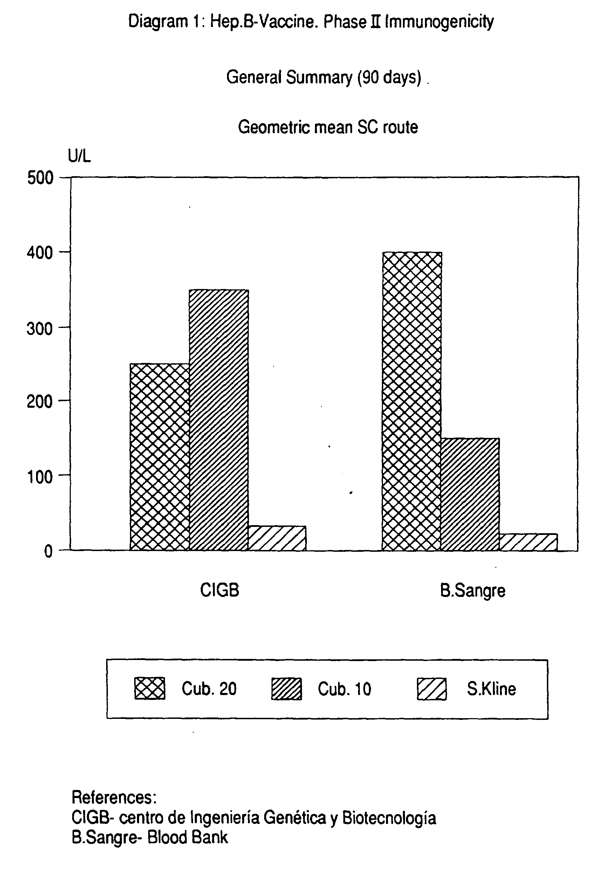

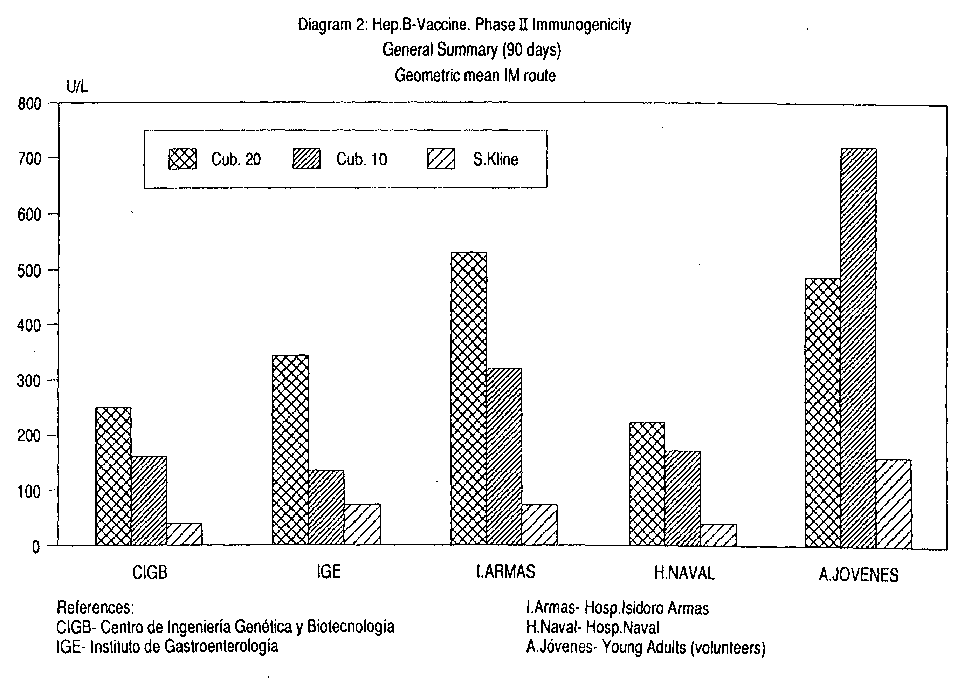

The final integration vector was obtained by subcloning the exact gene for HBsAg under the perfect promotor of the enzyme AOX1 (Fig. 7). The resulting clone, called pHAO112, contains the gene for HBsAg under the regulation signal of the AOX1 promotor and the termination signal of the GAP gene of S. cerevisiae, in which there is also a region of chromosomal DNA of P. pastoris necessary for homologous recombination with the yeast. By partial digestion and treatment with S1 nuclease of this plasmid, the SalI site was destroyed which was located between the 3' end of the HBsAg gene and the terminator of the enzyme glyceraldehyde 3-phosphate dehydrogenase. The resulting clone was called pSAO503 (Fig. 8), and in it was subcloned the 2.4 kb fragment which contains a fragment of the structural gene for the enzyme AOX1 as well as the non-coding 3' region which flanked it, which was obtained from the plasmid pCAO10. The resulting plasmid was called pVAO721 (Fig. 8), in which was cloned the 1.8 kb fragment which contains the his3 gene of S. cerevisiae, from the plasmid pPMC (Fig. 9).

The clone obtained, pTA0906 (Fig. 10), yielded the final integration plasmid which was used for transformation of P. pastoris strain MP36 (EP-A 0 438 200). The individuals who showed clear patterns of integration were used for evaluation of the levels of antigen expression. The strain selected for production of the antigen was called C226.

A deposit according to the Budapest Treaty was made of Pichia pastoris C226 [pTAO906] on 22 October 1990 with the Centraal Bureau voor Schimmelcultures (CBS) at Baarn, the Netherlands, deposit number CBS 450.90.

### EXAMPLE 2:

To obtain the synthesis of HBsAg, the transformed strain C226 is cultivated in the preinoculum stage in a saline medium, the composition of which is:

| | |
|---|---|
| K₂HPO₄ - | 5 g/l |
| MgSO₄ - | 4.6 g/l |
| NH₄SO₄ - | 22 g/l |
| CaCl₂ - | 0.5 g/l |
| glycerol - | 20 ml/l |
| vitamins 200 x - | 10 ml/l |
| trace elements 200 x - | 5 ml/l |

Growth takes place at a temperature of 30°C and pH 4.5 under conditions of aeration and agitation such as guarantee an oxygen partial pressure greater than 30% of the saturation value. The culture is grown between 10 and 12 hours.

At the inoculum stage there is a change of saline medium for a rich medium of the following composition:

| | |
|---|---|
| yeast extract | 1% |
| peptone | 2% |
| glycerol | 2% |
| trace elements 200x - | 10 ml/l |
| vitamins 200x - | 5 ml/l |

The culture is maintained for 10-12 hours under these conditions until a cell concentration of 10-12 g/l is reached (on the basis of dry matter) in order to inoculate the fermentation which is carried out in the same rich medium and under identical conditions of temperature and pH. The culture is maintained for 8-10 hours until a cell concentration of 10-12 g/l is reached (on the basis of dry matter).

From that moment begins a continuous addition of methanol in order to induce expression of the antigen which reaches approximately 3% of the total protein at 90-100 hours cultivation. This addition is carried out with increases at intervals according to the increase in cell concentration in the medium. At the end of this period begins a culture increase with a rich medium having the following composition:

| | |
|---|---|
| yeast extract | 3% |
| peptone | 6% |

This increase is maintained until the end of cultivation, which lasts 180-200 hours, increasing the specific growth rate fivefold, and raising the levels of expression. A result of this method was also that the protein expressed in this way is maintained in soluble form in over 80%, and this factor determines greater efficiency in the subsequent stages of purification as well as improved particulation of the antigen.

In the final stage of fermentation, a biomass concentration of 60 to 80 g/l (on the basis of dry matter) is obtained and the levels of antigen expression reach between 2 and 5% of the total protein.

On completion of the fermentation, the cells are collected by centrifugation in a continuous system and are stored at 4°C as a cream after washing with sterile water using the same centrifugation system.

### EXAMPLE 3:

In order to obtain a HBsAg preparation suitable for column chromatographic processing, the crude extract obtained by mechanical disruption of the biomass obtained by centrifugation of the culture of the yeast transformed with the gene encoding the antigen, was subjected to the following purification method.

The cellular concentration of the washed cream was adjusted to 100 g/l with distilled water and in order to obtain a rupture buffer the following substances were added:

| | |
|---|---|
| Trishydroxymethylaminomethane - | 12.1 g/l |
| sucrose - | 100 g/l |
| NaCl - | 17.5 g/l |
| EDTA - | 1.86 g/l |
| KSCN - | 2 91 g/l |

The pH was adjusted between 7.5 and 8.0 with 1N HCl.

This solution was homogenised for 5 minutes and subjected to mechanical rupture of the cells on a bed mill.

The disrupted cells were subjected to a process of clarification based on the stability of HBsAg under extreme pH conditions, under which numerous contaminating proteins of the host yeast were precipitated, while the antigen remained in solution.

Clarification of the rupture extract by precipitation of contaminants at acid pH was carried out by the following method:

Disrupted cells homogenate was cooled to a temperature between 0 and 4°C, under continuous agitation and recording of pH, and it was mixed rapidly, avoiding foam formation, with a solution of 1N HCl at 4°C, in a quantity sufficient to lower the pH to a range between 2.5 and 3. After agitation for five more minutes, the mixture was centrifuged in a continuous centrifuge cooled to 4°C, with a holding time of 45 minutes, keeping cold the receptacles from which the material was extracted and collected. The supernatant was restored to a pH between 7.5 and 8.5 immediately after centrifuging, under constant agitation and recording pH.

The pH of the clarified supernatant was lowered again to 4.0 and was homogenised for one hour approximately with a matrix of Celite (High Flow Supercell, Fluka), in an approximate proportion of 0.35 g of the antigen per kg of the matrix. The antigen was adsorbed onto the matrix and by centrifuging, filtration or decanting, the latter was separated from the unadsorbed material which was discarded. Then 2 or 3 washes of the antigen fixed on the bed were performed with a buffer solution having the following composition:

| | |
|---|---|
| KSCN - | 48.5 g/l |
| sucrose - | 100 g/l |
| Tris - | 2.42 g/l |
| EDTA - | 1.86 g/l |

The pH was adjusted to 4.0 with 1N HCl.

After the successive washes, desorption of the antigen from the matrix was performed, using an elution buffer, the composition of which was the following:

| | |
|---|---|
| Tris - | 2.42 g/l |
| EDTA - | 1.12 g/l |
| sucrose - | 100 g/l |

The pH was adjusted between 9.0 and 9.5 with 1N NaOH.

The material obtained by the elution was introduced into an AMICON type concentrator (Hollow-fiber) with a cartridge having a pore size of 0.1 µm in order to concentrate its initial volume by a factor of about 55. The whole operation was performed at 4°C.

In order to complete the purification of the HBsAg obtained some chromatographic steps were performed as stated below:

About 2 liter of the concentrated celite eluates were desalted by a gel filtration column of Sephadex G-25 medium, expanded and equilibrated in 20 mM Tris-HCl buffer, pH 8.0. Progress was controlled by the absorbance at 280 nm and by conductivity.

An immunoaffinity matrix was prepared which contained about 2 liter Sepharose CL-4B activated by CNBr and approximately 10 g of the monoclonal antibody CB-HEP1 (obtained at the CIGB according to example 4) bound thereto, with special characteristics which allow the selection of antigen populations having high immunogenicity. The immunoaffinity column packed with the above matrix was equilibrated in 20 mM Tris-HCl buffer, pH 8.0, and was injected with a quantity of the desalted celite eluate concentrate which contained the antigen for an approximate ratio of 0.1 mg HBsAg per ml bed. The flow rate was about 50 cm/h at ambient temperature. Washing was performed with an equilibration buffer and progress was controlled by the absorbance at 280 nm with graphic recording. The effluent was collected until it reached the base line on the chromatogram, then a buffer containing 20 mM Tris-HCl, NaCl 1N, pH 8.0 was passed to eliminate non-specific adsorption until the base level was obtained again.

The antigen was eluted with a buffer containing 20 mM Tris-HCl, 3M KSCN, 1M NaCl, pH 8.0 and the column was washed inmediately with excess equilibration buffer in the presence of 0.01% thimerosal at 4°C, in case the column remains out of use for a long time.

The HBsAg eluted from the affinity column was subjected to a heat treatment at 37°C during 2 hours in a water bath. Later the antigen was desalted in a Sephadex G-25 column equilibrated with 20 mM Tris-HCl, 3 mM EDTA, pH 8.0.

In a column which contained DEAE cellulose (Whatman DE-52) equilibrated in 20 mM Tris-HCl, 3 mM EDTA, pH 8.0 buffer, were injected about 3 liter of the desalted material from the immunoaffinity column and the process was performed at 4°C. Then, subsequent washes with the equilibration buffer containing 20 mM Tris-HCl, 3 mM EDTA, 50 mM NaCl and 0.05% Na-Deoxycolate were performed. The process was controlled by the absorbance at 280 nm with graphic recording. The effluent was collected until the base line was reached on the chromatogram. The HBsAg was recovered from the column using a buffer containing 20 mM Tris-HCl, 3 mM EDTA, 150 mM NaCl, pH 8.0, collecting the effluent until the base level was reached again.

The fraction which was obtained from the ion exchange column as described in the previous step, was concentrated in an Amicon Hollow Fiber System (DC-2), using a cartridge of cut-off of 100,000 daltons about 25 times. Then, 0.05 g/100 ml of Na-Deoxycolate were added to the concentrate. Incubation was performed for 1 hour at 4°C and later this fraction was applied onto a PWG 6,000 column, equilibrated in 20 mM Tris-HCl, 3 mM EDTA and 0.05% Na-Deoxycolate, pH 8.0. One main peak was obtained which contained the antigen highly particulated, free from monomers or dimers of the same molecule. This fraction was collected and desalted on a G-25 column equilibrated in PBS, pH 7.0. This final preparation of purified HBsAg was filtrated sterile using a membrane of 0.2 µm for further adjuvation in Aluminium Hydroxide.

As the purified antigen as referred to above must be developed as an injectable preparation, all strict measures to comply with the requirements laid down for preparations for parenteral administration were taken and the corresponding quality controls were carried out (Requirements for Hepatitis B vaccines made by recombinant DNA techniques, Requirements for Biological Substances No. 45, World Health Organization, Technical Report Series, No. 786, 1989).

In the following table are summarized the results obtained during the purification process of HBsAg, object of the present invention.

| STEP | VOL (1) | HBsAg (mg) (Determ. by ELISA) | Total proteins (g) | Specific activity (mg HBsAg det. ELISA/g of prot.) | Overall yield |
|---|---|---|---|---|---|
| Disrupted cells | 168 | 26 600 | 880 | 30.2- | |
| Acid crecipitation | 176 | 14 520 | 114 | 127.3 | 54% |
| Celite adsorption and concentration | 34.5 | 4 640 | 7.1 | 653.5 | 17% |
| Immunoaffinity and heat treatment | 9.5 | 3 120 | 3.2 | 975 | 11% |
| Ion exchange | 2.5 | 1 936 | 1.86 | 1040 | 7.2% |
| HPLC gel filtration | 0.54 | 1 500 | 1.42 | 1056 | 5.6 % |

### EXAMPLE 4

In order to obtain a monoclonal antibody (MAb) specific to HBsAg, 8-week-old Balb/c male mice were immunised with natural hepatitis B virus surface antigen obtained from human plasma (S. Krugman et al., J. Am. Med. Assoc., 1971, 217: 41). Fusion, cultivation and tracing of the hybrids were carried out according to the basic principles described by Kholer (The Technic of Hybridoma Production, Immunological Methods, 1981, Vol. I, Academic Press, 285-298). The spleen cells were hybridised with myeloma line SP2/0/Ag14 in a ratio of 10:1, using 50% polyethylene glycol 1500 (BDH), and seeded on plates with 96 wells (Costar), at a concentration of 100,000 cells per ml of medium and 100 µl per well. The selective culture medium used was RPMI 1640 (Gibco), which can be supplemented with 1 g/l NaHCO₃, 3µg/l HEPES, 2mM L-glutamine, 1 mM sodium pyruvate, 0.05 mM 2-mercaptoethanol, 10% newborn calf serum, 40 µg/ml gentamicin and 3% of human endothelial cell culture supernatant (HECS), (Astaldi et al., Methods of Enzymology, 1983, Vol. XCII, ed. J. Langone, Academic Press, 39-46), to which were added hypoxanthine up to 0.03 mM, thymidine up to 3 µM and aminopterin up to 0.4 µM.

From the third week of cultivation on, the supernatants were investigated for antibodies specific to HBsAg, using an indirect ELISA. Among the positive wells, No. 48 was selected for the high values obtained in repeated assays, and was cloned and recloned several times by limiting dilution. As the final clone there was selected 48/1/5/4, which secreted a MAb having the following immunochemical characteristics:
- It recognises in a specific manner both natural HBsAg and recombinant HBsAg produced in yeasts (HBsAgr). This was demonstrated in ELISA with both molecules.
- It recognises HBsAgr both in solid phase (ELISA) and in solution. It is possible to inhibit binding of the MAb to the antigen in solid phase by prior incubation with it in solution.
- No cross reactions are observed in ELISA on assaying with several molecules as coating. It also specifically recognises in a Western blot the band corresponding to HBsAg of a preparation of yeast which produces HBsAgr. This allows us to affirm that this MAb is highly specific to HBsAg and that the epitope recognised is of a continuous type.
- It recognises in an equivalent manner to the international standards of subtypes ay and ad, which indicates that it is directed against the determinant common to all subtypes of the virus.
- A special characteristic of this clone is the fact that it synthesises two types of heavy chains: one of subclass gamma 2b and the other of class M. The cells secrete both antibodies of class IgG 2b and pentameric IgM molecules which can be separated by gel filtration chromatography (Sephacryl S-300). This has been demonstrated by double radial immunodiffusion (O. Ouchterlony et al., 1973, Handbook of Experimental Immunology, vol. 1: 19.1) and polyacrylamide gel electrophoresis (N.K. Laemmli, Nature, 1970, 227: 680). Both antibodies are specific to HBsAg, which has been demonstrated by ELISA with conjugates specific to mouse IgG and IgM (Sigma).
- Experiments on coated PVC plates indicate that the MAb is capable of trapping HBsAg efficiently. A high percentage of it can be eluted subsequently, using solutions with a high molarity of potassium thiocyanate.

A deposit according to the Budapest Treaty was made of this hybridoma cell line on 26 November 1990 with the European Collection of Animal Cell Cultures, Salisbury, United Kingdom, deposit number ECACC 90 112 606.

For the production of this monoclonal antibody, pure preparations of it are obtained from ascites fluid extracted after inoculation of 3 million hybrid cells in the peritoneal cavity of Balb/c mice preinjected 10 days beforehand with mineral oil by the same route. The ascites collected by repeated puncture is centrifuged at 500 g, delipidised with chloroform 1:1, and dialysed with phosphate-buffered saline (PBS) .

For purification there is used a precipitation with 50% NH₂SO₄ for 2 hours at 4°C, centrifuging at 4000 r.p.m. for 30 minutes, two additional washes of the precipitate with the same concentration of NH₂SO₄ and desalting in Sephadex G-25 (Pharmacia). The desalted specimen is fractionated by affinity chromatography in protein A Sepharose CL4B (Pharmacia), the instructions suggested by the manufacturer being followed.

The purified antibodies are bound to matrices of Sepharose CL4B activated with cyanogen bromide according to the method proposed by the manufacturer. Per ml of gel, 5 mg of MAb are used.

### EXAMPLE 5

In order to obtain a vaccine preparation from the pure recombinant antigen, with high homogeneity and degree of particulation, the latter was mixed with a sterile aluminium hydroxide solution.

Following this formula, several batches were prepared for carrying out the necessary tests for characterisation of this product. Then the results obtained were detailed in the evaluation of three consecutive batches of this preparation.

| RESULTS OBTAINED | | | | |
|---|---|---|---|---|
| ANALYSIS PERFORMED | | BATCH 1 | BATCH 2 | BATCH 3 |
| Polyacrylamide gel electrophoresis with SDS, stain Ag/Coomassie and Western blot (WB) | | A band of 24 kDa | A band of 24 kDa | A band of 24 kDa |
| Specific protein (*) µgAg (ELISA)/mg protein (Lowry) | | > 3400 | > 3700 | > 1250 |
| Contamination with proteins of host yeast (DOT and WB antiserum to yeast proteins) | | < 5% | < 3% | < 3% |
| Electron immunomicroscopy and particle size distribution | | 22 nm particles | | |
| Nucleic acid content (random hybridisation with hexanucleotides) | | 7 pg/dose | < 5 pg/dose | < 3 pg/dose |
| Potency test (100 mice) >50% seroconversion after single minimum dose of (µgAg) : Cuban vaccine (1) test, commercial vaccine (2) control | (1) | 0.62 | 1.25 | < 0.31 |
| | (2) | - | 1.25 | 1.25 |
| Content of mouse Ig's | (1) | Not detectable | | |
| (ELISA with mouse serum) | (2) | < 50 pg/dose | < 20 pg/ dose | < 50 pg/ dose |
| Lipopolysaccharide content | (1) | Not detectable | | |
| by determination of KDO groups | (2) | <0.8 µg/dose | | |
| Immunogenicity (12 rabbits) seroconversion 40 µg 30 days | | - | 100% | 86% |
| after one dose 20 µg 11 days | | - | 88% | 44% |
| after two doses 20 and 40 µg | | - | 100% | 100% |
| Safety test with 10 doses for humans, mice and guinea pigs | | Satisfactory | | |
| Sterility test | | Satisfactory | | |
| Pyrogen determination (LAL) | | - | <8 EU/ml | <6 EU/ml |

| | | | | |
|---|---|---|---|---|
| (*) Specific protein values greater than 1000 µg/ml are due to underestimation of immunogenic protein by Follin. | | | | |

### EXAMPLE 6

To study the immunogenicity characteristics of the hepatitis B virus surface antigen obtained by the recombinant DNA route in yeasts as described in the above examples, a protocol was carried out for preclinical tests on groups of volunteers, after demonstrating that the preparation complies with the requirements of the regulations for use in human beings by parenteral administration (Requirements for Hepatitis B vaccines made by recombinant DNA techniques, Requirements for Biological Substances No. 45, World Health Organization, Technical Report Series, No. 786, 1989). These studies were conducted by a Committee for Evaluation created for this purpose by the Ministry of Public Health of Cuba, which issued a report of results which is given in summary form below:

The Committee for Evaluation of the Cuban recombinant vaccine against hepatitis B of the Cuban Ministry of Public Health has drawn up a complete final report of a prototype phase II study for evaluation of the immunogenicity of the Cuban vaccine of recombinant DNA origin of hepatitis B virus surface antigen (rec-HBsAg) in yeast,

This report which is summarised here is a typical controlled double-blind experiment designed to compare the behaviour of the Cuban rec-HBsAg vaccine with an established, commercially available vaccine of the same type which is approved and registered in nearly one hundred countries (Smith & Kline).

Out of a total of 123 persons who signed a document as a record of their consent to voluntary participation in the study, 35 were excluded for different reasons and there were selected 88 clinically healthy persons free from markers of the virus (HBsAg, HBeAg, anti-HBsAg, anti-HBcAg), with normal values of transaminases (ALT). The sample mainly consisted of young adults of both sexes, aged between 20 and 34, uniformly distributed in three groups of 31, 30 and 27 persons, who were inoculated with 3 doses (1 ml antigen adsorbed on aluminium hydroxide) of 20 µg of the Cuban vaccine (CV20), 10 µg of the Cuban vaccine (CV10) and 20 µg of the commercial vaccine (SK20), respectively, on days 0, 30 and 60.

Blood samples were taken on the same days before the vaccination and on days 15, 75 and 90 intra or post-vaccination. 45% of the total group of persons were inoculated subcutaneously and the rest intramuscularly (deltoid muscle), without significant differences between the groups relating to age, sex or methods of administration.

On day 90 after completing the whole schedule of vaccination, in 29 of those vaccinated and assessed out of the 31 who received CV20, 100% showed antibody titres greater than the agreed minimum protective level (MPL) of 10 IU/litre, with a geometric mean titre (GMT) of 239.9 IU/litre.

In 26 of those vaccinated and assessed out of the 30 who received CV10, 100% showed antibody titres greater than the MPL with a GMT of 218.4 IU/litre, while out of 26 persons vaccinated and assessed of the 27 who received SK20, 23 (88.5%) had antibodies with a GMT of 43.9 IU/litre, but only 21 (80.8%) exceeded the MPL.

On comparing the results of day 90 with those of day 75 (15 days after the third dose), both Cuban vaccines CV20 and CV10 maintained a maximum rate of seroconversion (100%) and increased to 100% the number of those vaccinated with titres greater-than the MPL, whereas with SK20 the number of those who responded decreased from 96.3% to 88.5%, but the number of persons with titres greater than the MPL increased from 70.4% to 80.8%. These data are still significantly worse (p) for SK20 than for both Cuban preparations.

It can be concluded, consequently, that out of a total of 81 persons vaccinated and assessed who completed the vaccination schedule (92% of those who received the first dose), seroconversion was produced in 78 (96.3%), out of whom 55 (100%) had received one or other of the Cuban vaccines (all of them with titres greater than the MPL) and 23 (88.5%) had received the commercial vaccine, 21 (80.8%) of them with titres greater than the MBL. Based on these results which are significantly in favour of the Cuban vaccine, it was decided to extend this study to a larger group of persons selected in the manner described above and grouped at different institutions of the national health service.

The results of this second phase are given in summary form in graphs 1 and 2. In these graphs can be seen the levels of response for both preparations (Cuban vaccine v. SK) using the methods of intramuscular (IM) and subcutaneous (SC) immunisation.

### DRAWINGS:

The following abbreviations are used in the drawings:
- B: BamHI
- N: NcoI
- S: SalI
- E_{I}: EcoRI
- E_{V}: EcoRV
- BgII: BglII
- C: ClaI
- CIP: Calf Intestine Phosphatase
- Klenow: DNA polymerase I Large Fragment

### SEQUENCE LISTING

SEQ ID NO:1.
   SEQUENCE TYPE: nucleotide
   SEQUENCE LENGTH: 19 nucleotides GCCATGGAGA ACATCACAT
SEQ ID NO : 2
   SEQUENCE TYPE: nucleotide
   SEQUENCE LENGTH: 25 nucleotides GGTCGACGTT TAAATGTATA CCCAG
SEQ 10 NO:3
   SEQUENCE TYPE: nucleotide
   SEQUENCE LENGTH: 15 nucleotides GTATCACGAG GCCCT
SEQ ID NO:4
   SEQUENCE TYPE: nucleotide
   SEQUENCE LENGTH: 24 nucleotides

CGCCATGGTT TCGAATAATT AGTT

## Claims

1. Recombinant hepatitis B surface antigen, obtainable by a process comprising
growing cells of Pichia pastoris strain C226 [pTAO906], CBS450.90, containing a gene encoding said hepatitis B surface antigen under conditions resulting in its expression;
lysing the cells in the presence of a buffer comprising a chaotropic agent, sucrose and ethylene diamino tetraacetic acid (EDTA);
precipitating contaminants at an acid pH;
subjecting the antigen preparation to an acid adsorption and alkaline desorption treatment on a matrix of diatomaceous earth;
subjecting the antigen preparation to immunoaffinity chromatography using the Hepatitis B surface antigen-specific monoclonal antibody CB-HEP1 produced by hybridoma cell line ECACC 90 112 606;
subjecting the eluted antigen to a heat treatment at a temperature of from 30 to 40°C;
washing the antigen in an anion exchange column with detergent; and
subjecting the eluted antigen to HPLC in the presence of a detergent.

2. Vaccine composition for immunizing against Hepatitis B Virus, comprising recombinant Hepatitis B surface antigen as claimed in claim 1 in an amount effective for vaccination, and at least one carrier, diluent or adjuvant appropriate for use in a vaccine composition.

3. Use of Pichia pastoris strain C226 [pTA0906], CBS450.90, in a method as defined in claim 1.

4. Use of monoclonal antibody anti-HBsAg CB-HEP1, produced by hybridoma cell line ECACC 90 112 606, in a method as defined in claim 1.

## Patentansprüche

1. Rekombinantes Hepatitis B-Oberflächenantigen, das erhalten werden kann nach einem Verfahren, umfassend:
Züchten von Zellen des Stammes Pichia pastoris C226 [pTA0906], CBS450.90, die ein Gen enthalten, das dieses Hepatitis B-Oberflächengen codieren kann, bei Bedingungen, die zu dessen Expression führen;
Auflösen der Zellen in Gegenwart eines Puffers, der ein chaotropes Mittel, Sacccharose und Ethylendiamintetraessigsäure (EDTA) umfaßt;
Fällen von Verunreinigungen bei einem sauren pH-Wert;
Unterziehen des Antigenpräparats einer sauren Adsorptions- und alkalischen Desorptionsbehandlung über einer Kieseleerdematrix;
Unterziehen des Antigenpräparats der Immunoaffinitätschromatographie unter Verwendung des für das Hepatitis B-Oberflächenantigen spezifischen monoklonalen Antikörpers CB-HEP1, der von der Hybridomzellinie ECACC 90 112 606 produziert worden ist;
Unterziehen des eluierten Antigens einer Wärmebehandlung bei einer Temperatur von 30 bis 40°C;
Waschen des Antigens in einer Ionenaustauschsäule mit einem Detergens; und
Unterziehen des eluierten Antigens der HPLC in Gegenwart eines Detergens.

2. Impfstoffzusammensetzung für die Immunisierung gegenüber dem Hepatitis B-Virus, umfassend ein rekombinantes Hepatitis B-Oberflächenantigen nach Anspruch 1 in einer für das Impfen wirksamen Menge und mindestens einen Träger, ein Verdünnungsmittel oder ein Adjuvans, die für die Verwendung in einer Impfstoffzusammensetzung geeignet sind.

3. Verwendung des Stammes Pichia pastoris C226 [pTAO906], CBS450.90 bei einem Verfahren, wie es in Anspruch 1 angegeben ist.

4. Verwendung des monoklonalen Antikörpers Anti-HbsAg CB-HEP1, der von der Hybridomzellinie ECACC 90 11 2 606 produziert worden ist, bei einem Verfahren, wie es in Anspruch 1 angegeben ist.

## Revendications

1. Antigène de surface de l'hépatite B recombinant, pouvant être obtenu par un procédé comprenant les étapes comprenant :
- mettre en culture des cellules de la souche *Pichia pastoris* C226 [pTAO906], CBS450.90 contenant un gène codant ledit antigène de surface de l'hépatite B dans des conditions permettant son expression ;
- lyser les cellules en présence d'un tampon comprenant un agent chaotropique, du saccharose et de l'acide éthylène diamine tétraacétique (EDTA) ;
- précipiter les contaminants à un pH acide ;
- soumettre la préparation d'antigène à un traitement d'adsorption acide, et de désorption alcaline sur une matrice de terre diatomée ;
- soumettre la préparation d'antigène à une chromatographie d'immunoaffinité en utilisant l'anticorps monoclonal CB-HEP1 spécifique de l'antigène de surface de l'hépatite B produit par la lignée cellulaire d'hybridome ECACC 90 112 606,
- soumettre l'antigène élué à un traitement thermique à une température allant de 30 à 40°C,
- laver l'antigène sur une colonne échangeuse d'anions avec un détergent ; et
- soumettre l'antigène élué à une CLHP en présence d'un détergent.

2. Composition de vaccin pour l'immunisation contre le virus de l'hépatite B, comprenant un antigène de surface de l'hépatite B recombinant selon la revendication 1 dans une quantité efficace pour une vaccination, et au moins une charge, un diluant ou un adjuvant approprié à une utilisation dans une composition de vaccin.

3. Utilisation de la souche *Pichia pastoris* C226 [pTAO906], CBS450.90, dans un procédé tel que défini dans la revendication 1.

4. Utilisation d'un anticorps monoclonal anti-HBsAg CB-HEP1, produit par la lignée cellulaire d'hybridome ECACC 90 112 606, dans un procédé tel que défini dans la revendication 1.
